# EUROPÄISCHE PATENTSCHRIFT

(11) **EP 2 674 127 B1**
(45) Veröffentlichungstag und Bekanntmachung des Hinweises auf die Patenterteilung: **17.12.2014**
(21) Anmeldenummer: 12004462.3
(22) Anmeldetag: 13.06.2012
(51) Int. Cl.: A61K 6/02, A61C 8/00

(54) **Implantat**
Implant
Implant

(43) Veröffentlichungstag der Anmeldung: 18.12.2013
(73) Patentinhaber: maxon motor ag, 6072 Sachseln (CH)
(72) Erfinder: Buurlage, Thorsten, 69517 Gorxheimertal (DE)
(74) Vertreter: Grünecker, Kinkeldey, Stockmair & Schwanhäusser

(56) Entgegenhaltungen:
- EP-A1- 2 283 793
- US-A- 5 915 967
- US-A1- 2005 147 943
- US-A1- 2010 036 502
- US-A1- 2011 070 557

## Beschreibung

Die vorliegende Erfindung betrifft ein Implantat, insbesondere ein Zahnimplantat, mit einem Grundkörper und einem an einem äußeren Ende des Grundkörpers angeordneten Gewinde, wobei in dem Bereich des Grundkörpers der kein Gewinde aufweist, ringförmige Stege und/oder Vertiefungen ausgebildet sind.

Der Einsatz von Implantaten zur Behebung von Beschädigungen an Knochen, Gelenken oder an Zähnen ist schon seit langem bekannt. So werden beispielsweise implantierbare Schrauben oder implantierbare Verbindungsstücke zur Stabilisierung von gebrochenen Knochen oder in künstlichen Gelenken eingesetzt. Auch bei Totalverlust eines oder mehrerer Zähne ist es mittlerweile üblich, ein Zahnimplantat zu empfehlen. Dabei wird ein Grundkörper des Implantats im Kiefer verankert, auf dem Grundkörper kann mit Hilfe verschiedener Mittel die Suprakonstruktion, also beispielsweise eine Brücke oder eine Krone befestigt werden.

So wird beispielsweise in der WO 2007/090529 A1 ein zum Einwachsen in einen Knochen bestimmtes Implantat beschrieben. Das Implantat umfasst einen Implantatkörper, einen Implantatpfosten und eine Suprakonstruktion. Um den Implantatkörper gut im Kieferknochen zu verankern, ist der Implantatkörper mit einem Gewinde versehen, das sich über die komplette Länge des Implantatkörpers erstreckt. Nach Einwachsen des Implantatkörpers im Kieferknochen wird der Implantatpfosten in dem Implantatkörper befestigt, auf dem Implantatpfosten wird die Suprakonstruktion aufgebracht.

Nachteilig an diesem Implantat ist, dass das Implantat durch das über die gesamte Länge des Implantatkörpers verlaufende Schraubgewinde im Knochen befestigt wird. Dazu wird im Knochen eine Bohrung und das Schneiden eines Gewindes benötigt. Ein präzises Einsetzen ist daher schwierig. Insbesondere das Eindrehen ist in der Praxis langwierig und auf begrenztem Raum diffizil.

Es sind auch Implantate bekannt, die mittels eines Presssitzes im Kiefer befestigt werden. Ein solcher Presssitz ist aber aufgrund des Druckes auf den Knochen und der fehlenden Präzision nicht besonders vorteilhaft.

Die DE 197 18 175 A1 zeigt ein weiteres Zahnimplantat mit einem in den Kieferknochen implantierbaren Grundkörper. Diesem Implantat liegt die Aufgabe zugrunde, eine stabile Verankerung des Grundkörpers mit einer geringen Beschädigung des Knochengewebes des Patienten zu ermöglichen. Dazu ist vorgesehen, dass der Grundkörper an seinem inneren Ende, d.h. dem zuerst in den Kieferknochen eingebrachten Ende, einen ersten Abschnitt aufweist, an den sich ein zweiter Abschnitt anschließt, in dem der Außendurchmesser in Richtung zu dem äußeren Ende des Implantatgrundkörpers zunimmt. An diesen zweiten Abschnitt schließt sich ein dritter Abschnitt an, der mit einem selbstschneidenden Außengewinde versehen ist. Der Außendurchmesser des ersten Abschnitts ist kleiner als der Außendurchmesser des zweiten und des dritten Abschnitts. Auch bei diesem Implantat ist ein relativ langer Gewindeabschnitt vorgesehen. Durch die Gewindegänge und deren Kerbwirkung wird die Stabilität des Implantats herabgesetzt. Auch hier ist das Eindrehen langwierig und diffizil.

Aus der US 5,915,967 ist bereits ein Zahnimplantat bekannt, das an einem Ende ein Gewinde aufweist, und in dem Bereich, an dem kein Gewinde ausgebildet ist, mit ringförmigen Stegen und Rillen versehen ist. Ähnliche Zahnimplantate sind aus der EP 2 283 793 A1, der US 2010/036502 A1, der US 2005/147943 A1 und der US 2011/070557 A2 bekannt.

Es ist daher die Aufgabe der vorliegenden Erfindung, ein Implantat bereitzustellen, das die Nachteile der aus dem Stand der Technik bekannten Implantate vermeidet und insbesondere ein schnelles und präzises Einsetzen des Implantates mit geringem Druck auf den Knochen ermöglicht. Ferner soll das Implantat so ausgebildet sein, dass das Risiko der Lockerung und somit des Verlustes des im Knochen eingesetzten Implantates minimiert wird.

Hierzu ist erfindungsgemäß vorgesehen, dass die Länge des Bereichs des Grundkörpers, an dem das Gewinde angeordnet ist, etwa einem Zehntel der Länge des gesamten Grundkörpers entspricht, das Gewinde mehrgängig ausgebildet ist und die Länge der einzelnen Gewindegänge kleiner ist als der Umfang des Grundkörpers und bevorzugterweise etwa ein Viertel des Umfangs des Grundkörpers beträgt.

Durch das kurze Gewinde wird das Gewindeschneiden und das Eindrehen des Gewindes in die Bohrung über die Gesamtlänge des Implantats vermieden. Wie bereits beschrieben, ist insbesondere das Eindrehen langwierig und auf begrenztem Raum diffizil. Bei dem erfindungsgemäßen Implantat wird der Grundkörper lediglich in eine Öffnung bzw. Bohrung im Knochen eingesteckt und durch kurzes Eindrehen arretiert. Dies geht besonders einfach, wenn das Gewinde als Schneidgewinde oder Furchgewinde ausgebildet ist. Hierdurch wird die Anzahl der Arbeitsschritte verringert, das Implantat lässt sich sehr präzise in die Bohrung einführen. Durch die fehlenden Gewindegänge und die somit auch fehlende Kerbwirkung wird das Implantat stabiler. Durch die ringförmigen Stege und/oder Vertiefungen wird verhindert, dass sich das Implantat lockert und wieder selbst aus der Bohrung im Knochen herausschrauben kann. Dabei müssen die ringförmigen Stege bzw. Vertiefungen nicht geschlossen um den Umfang des Implantats verlaufen, sondern können auch Unterbrechungen aufweisen. Wichtig ist, dass die Steigung der ringförmigen Linie, auf der die Stege bzw. Vertiefungen angeordnet sind, bei einem kompletten Umgang um den Umfang des Grundkörpers Null ergibt. Die ringförmigen Stege bzw. Vertiefungen sind somit nicht wendelförmig bzw. schraublinienförmig, womit das unerwünschte Herausschrauben des Implantats aus der Öffnung im Knochen vermieden wird. Es hat sich gezeigt, dass diese Länge für den Gewindebereich ausreichend ist, um das Implantat nach dem Einsetzen in den Knochen zu fixieren. Die Belastung auf den Knochen wird dabei sehr gering gehalten. Ein kurzes Andrehen reicht, um das Implantat wie gewünscht zu platzieren und zu befestigen. Damit wird ein guter Halt des Implantats im Knochen trotz der geringen Länge des Gewindes ermöglicht. Durch eine kurze Drehung, bevorzugt über ein Viertel bis einen halben Umfang des Grundkörpers, wird das Implantat dann im Knochen arretiert.

Gemäß einer bevorzugten Ausführungsform der Erfindung kann vorgesehen werden, dass die ringförmigen Stege und/oder Vertiefungen geschlossen sind. Dadurch wird die Wahrscheinlichkeit eines Herauswanderns des Implantats aus dem Knochen weiter reduziert.

Es kann auch vorgesehen werden, dass der Bereich des Grundkörpers, der kein Gewinde aufweist, als Schiebesitz ausgebildet ist. Unter dem Begriff Schiebesitz ist hier zu verstehen, dass der Grundkörper in dem gewindefreien Bereich keine Elemente aufweist, die über die Oberfläche des Grundkörpers vorstehen. Die Stege weisen also maximal die selbe Höhe auf wie die restliche Oberfläche des Grundkörpers. Dadurch ist ein leichtes Einführen des Implantates im Bereich des Schiebesitzes möglich, das Implantat wird lediglich in die Bohrung im Knochen eingesteckt. Es tritt dabei keine Belastung auf den Knochen auf, wie dies beispielsweise bei einem Presssitz der Fall wäre. Zur Befestigung des Implantats im Knochen reicht es aus, das Implantat in die Bohrung einzuschieben und das Implantat anschließend kurz in der Bohrung anzudrehen, so dass das Gewinde in der Bohrung greift. Dadurch wird ein sehr präziser Sitz des Implantats ermöglicht. Vorzugsweise ist der Grundkörper im Bereich des Schiebesitzes zylindrisch ausgebildet.

In noch einer weiteren Ausführungsform kann vorgesehen werden, dass die ringförmigen Stege und/oder Vertiefungen zumindest an dem von dem Gewinde abgewandten Ende des Grundkörpers angeordnet sind. Es handelt sich dabei also um das Ende, das zuerst in den Knochen eingebracht wird. Bei einem Zahnimplantat handelt es sich somit um das untere, im Kieferknochen angeordnete Ende. Es wird dadurch eine feste Verankerung des Zahnimplantats im Wurzelbereich ermöglicht.

Es kann ferner auch vorgesehen werden, dass die ringförmigen Vertiefungen benachbart zueinander liegen, so dass sie die Stege ausbilden. Zwischen den ringförmigen Vertiefungen bleibt also Material an der Oberfläche des Grundkörpers des Implantats stehen, das Stege ausbildet. Die ringförmigen Vertiefungen bilden gleichzeitig Hinterschneidungen aus, an die Knochenmaterial anwächst und das Implantat so in dem Knochen fixiert.

Eine vorteilhafte Ausgestaltung kann vorsehen, dass die Vertiefungen als ringförmige Rillen und/oder als konkave Einbuchtungen ausgebildet sind. Die ringförmigen Rillen führen zu einer gleichförmigen Verankerung des Grundkörpers im Kieferknochen am gesamten Umfang. Die konkaven Einbuchtungen, die beispielsweise wie die Oberfläche eines Golfballes ausgebildet sein können, sorgt ebenfalls für einen guten gleichmäßigen Halt des Implantats im Knochen, wenn der Knochen an das Implantat angewachsen ist. Vorzugsweise sind die Vertiefungen gleichmäßig voneinander beabstandet.

Vorteilhafterweise kann vorgesehen werden, dass die konkaven Einbuchtungen auf ringförmigen Linien liegen. In diesem Fall bilden auch die konkaven Einbuchtungen, die auf zwei nebeneinander liegenden ringförmigen Linien liegen, zwischen sich jeweils ringförmige Stege aus, die einen guten Halt des Implantats garantieren.

Es kann auch vorgesehen werden, dass die konkaven Einbuchtungen auf zwei nebeneinander liegenden ringförmigen Linien versetzt zueinander angeordnet sind. Somit wird eine dichte Anordnung der konkaven Einbuchtungen erreicht und ein guter Halt des Implantates im Knochen ermöglicht.

Noch eine weitere Ausführungsform sieht vor, dass der Bereich des Grundkörpers, an dem das Gewinde angeordnet ist, kürzer ist als der Bereich des Grundkörpers, an dem kein Gewinde angeordnet ist. Dadurch wird eine geringe Belastung des Knochens ermöglicht, trotzdem wird ein guter Halt des Implantats im Knochen erzielt.

Es kann ferner auch vorgesehen werden, dass das innere Ende des Grundkörpers abgerundet ist. Es handelt sich dabei um das Ende, das in der Bohrung im Knochen angeordnet ist. Dadurch ist eine gute Kräfteverteilung möglich, das Implantat kann einfach in die Bohrung eingeführt werden, insbesondere dann, wenn sich der Grundkörper zu diesem inneren Ende hin verjüngt.

In einer weiteren Ausgestaltung kann vorgesehen werden, dass das Implantat aus Keramik, insbesondere Zirkoniumoxid oder Aluminiumoxid oder aus einer Kombination dieser beiden Oxidkeramiken, besteht. Dadurch wird ein stabiles zahnfarbenes Implantat zur Verfügung gestellt.

In noch einer weiteren vorteilhaften Ausführungsform kann vorgesehen werden, dass die Oberfläche des Grundkörpers Mikrorauhigkeiten aufweist. Durch diese Mikrorauhigkeiten wird das Anwachsen von Knochen an das Implantat verbessert.

Im Folgenden wird die Erfindung anhand von Figuren näher erläutert.

Es zeigen:
- Fig. 1: erste Ausführungsformen eines Dentalimplantats
- Fig. 2: zweite Ausführungsform eines Dentalimplantats und
- Fig. 3: dritte Ausführungsform eines Dentalimplantats.

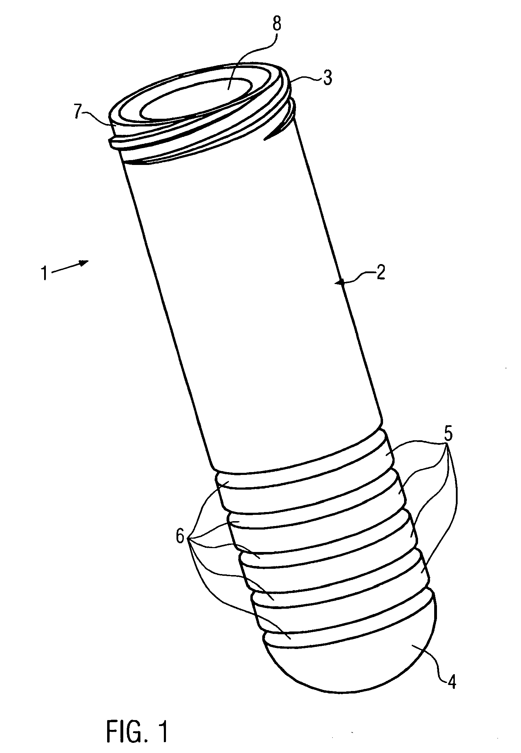

Figur 1 zeigt eine erste Ausführungsform des Implantats als Dentalimplantat 1. Es ist allerdings auch eine Ausführung als implantierbare Schraube oder als implementierbares Verbindungsstück denkbar. Das Dentalimplantat 1 umfasst einen Grundkörper 2, der im Wesentlichen zylindrisch ausgebildet ist. An seinem äußeren Ende, d.h., an dem Ende, das im eingesetzten Zustand des Dentalimplantats in Richtung des Mundraumes zeigt, ist an dem Grundkörper 2 ein Gewinde 3 angeordnet. Das entgegengesetzte Ende des Grundkörpers 2, d.h. das innere Ende, das im eingesetzten Zustand des Grundkörpers 2 im Kieferknochen angeordnet ist, ist abgerundet ausgebildet. In Figur 1 ist das innere Ende 4 halbkugelförmig. Zwischen dem inneren Ende 4 und dem Gewinde 3 sind an der Oberfläche des Grundkörpers 2 ringförmige Stege 5 und ringförmige Vertiefungen 6 ausgebildet. Die Vertiefungen 6 haben die Form von kreisförmig um den Umfang des Grundkörpers 2 verlaufende Rillen. Die Rillen 6 sind benachbart zueinander angeordnet, so dass zwischen den Rillen 6 Material an der Oberfläche des Grundkörpers 2 stehen bleibt und die Stege 5 ausbildet. Die Rillen 6 und somit auch die Stege 5 sind nahe des inneren Endes 4 angeordnet und erstrecken sich über etwa ein Drittel bis die Hälfte des Grundkörpers 2. Der restliche Bereich des Grundkörpers 2 von den Rillen 6 bis zum Gewinde 3 weist eine glatte Oberfläche auf. Somit weist der gesamte Grundkörper 2 außerhalb des Bereichs, in dem das Gewinde 3 angeordnet ist, keine Elemente auf, die über die Oberfläche des Grundkörpers 2 hervorstehen. Somit ist der Bereich des Grundkörpers 2, an dem kein Gewinde angeordnet ist, als Schiebesitz ausgebildet. Das Dentalimplantat 1 kann dann einfach in eine zylindrische Bohrung im Kieferknochen eingeschoben werden, ohne dass Spannungen oder Druck auf das umliegende Gewebe ausgeübt wird.

Das Gewinde 3 ist vorzugsweise als Schneidgewinde oder Furchgewinde ausgebildet. Das Gewinde 3 ist, wie bereits beschrieben, am äußeren Ende 7 des Implantatgrundkörpers 2 angeordnet. Das Gewinde 3 erstreckt sich über maximal ein Zehntel der Gesamtlänge des Grundkörpers 2. Das Gewinde 3 ist mehrgängig ausgebildet und umfasst somit zwei oder mehr Gewindegänge. Dadurch wird auch bei der kurzen Länge des Gewindes 3 ein sicherer Halt und eine präzise Befestigung des Dentalimplantats 1 im Kiefer ermöglicht. Durch die durch die Rillen 6 ausgebildeten Hinterschneidungen im Implantatgrundkörper 2 wird das Anwachsen von Kieferknochen an das Dentalimplantat 1 erleichtert. Es kann zudem vorgesehen werden, dass die Oberfläche des Implantats 1 Mikrorauhigkeiten aufweist, die nochmals zu einem verbesserten Anwachsen von Kieferknochen an das Implantat führen. Durch die kurze Länge des Gewindes 3 kann das Dentalimplantat mittels einer kurzen Drehung, vorzugsweise einer Drehung um einen halben oder Viertelumfang des Dentalimplantats 1 im Kieferknochen arretiert werden.

Vorzugsweise ist das Implantat aus Keramik, insbesondere aus Zirkoniumoxid, gefertigt. Das Implantat kann einteilig oder auch zweiteilig ausgebildet werden. Bei der einteiligen Ausbildung erfolgt die Befestigung der Suprakonstruktion, also einer Krone oder einer Brücke, direkt auf dem im Kieferknochen eingesetzten Dentalimplantat. Bei einer zweiteiligen Ausbildung wird ein Abutment auf dem Grundkörper befestigt, die Suprakonstruktion wird an dem Abutment fixiert. Figur 1 ist als eine zweiteilige Ausführung dargestellt. Am inneren Ende 7 des Grundkörpers 2 ist eine Bohrung 8 angeordnet, in der ein Abutment angebracht werden kann.

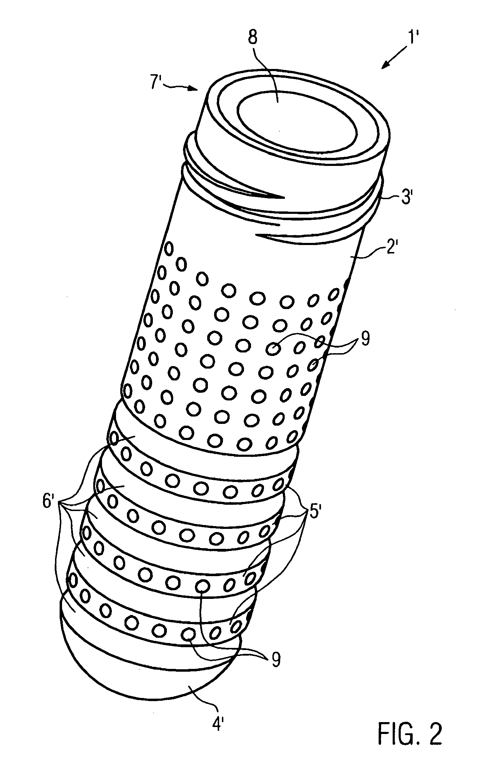

Figur 2 zeigt eine weitere Ausführungsform eines erfindungsgemäßen Dentalimplantats 1'. Dieses Dentalimplantats ist im Wesentlichen identisch zu dem Dentalimplantat aus Figur 1 aufgebaut. Im Folgenden wird auf die Unterschiede hingewiesen. Das Dentalimplantat 1' weist ebenfalls einen Grundkörper 2' auf. Am äußeren Ende 7' des Grundkörpers 2' ist das Gewinde 3' angeordnet. Auch dieses Gewinde 3' ist mehrgängig ausgebildet. Die Länge des Bereichs des Grundkörpers 2', an dem das Gewinde 3' angeordnet ist, entspricht auch hier maximal einem Zehntel der Gesamtlänge des Implantatgrundkörpers 2'.

Das innere Ende 4' des Grundkörpers 2' ist wieder abgerundet, vorzugsweise halbkugelförmig. Ausgehend von dem inneren Ende 4' des Grundkörpers 2' sind Vertiefungen in der Oberfläche des Grundkörpers 2' ausgebildet. Diese Vertiefungen haben die Form von kreisförmigen Rillen 6', die um den Umfang des Grundkörpers 2' verlaufen und geschlossene Ringe ausbilden. Es wäre aber auch möglich, dass die Rillen unterbrochen sind und schräg auf der Oberfläche des Grundkörpers 2' verlaufen, solange die Steigung einer Rille bei einem kompletten Umgang um den Umfang des Grundkörpers 2' Null ergibt. Dies bedeutet, dass die Rillen 6' keine Wendel-oder Schraubenform haben und somit ein Herausschrauben des Implantats nach Einsetzen in den Kieferknochen verhindert wird. Auch hier sind die Rillen 6' benachbart zueinander angeordnet, so dass zwischen zwei Rillen 6' Stege 5' an der Oberfläche des Grundkörpers 2' stehen bleiben. Die Rillen 6' bilden somit Hinterschneidungen im Grundkörper 2' aus, in die Kieferknochen anwachsen können. Um das Anwachsen von Kieferknochen an da Implantat 1' weiter zu erleichtern, sind an der Oberfläche des Grundkörpers 2' zusätzlich konkave Einbuchtungen 9 ausgebildet. Diese konkaven Einbuchtungen 9 sind sowohl auf den Stegen 5' als auch in dem Bereich des Grundkörpers 2' angeordnet, in dem weder Rillen 6' noch Gewinde 3' angeordnet ist. Vorzugsweise haben diese konkaven Einbuchtungen 9 die Form, wie die Oberfläche eines Golfballes (Dimple). Wie das in Figur 1 gezeigte Dentalimplantat 1 kann auch das Dentalimplantat 1' Mikrorauhigkeiten an der Oberfläche aufweisen, um das Anwachsen von Kieferknochen weiter zu erleichtern. Zudem ist der Bereich des Grundkörpers 2', in dem kein Gewinde angeordnet ist, als Schiebesitz ausgebildet. Das heißt, dass dieser Bereich keine über die eigentliche Oberfläche des Grundkörpers 2 vorstehenden Elemente aufweist, so dass der Grundkörper 2 einfach in eine Bohrung im Kieferknochen eingesetzt werden kann, ohne viel Druck auf den Knochen aufzubauen. Am inneren Ende 7' des Dentalimplantats 1 ist wiederum eine Bohrung 8 zur Aufnahme eines Abutments angebracht. Die konkaven Einbuchtungen 9 des Dentalimplantates 1 in Figur 2 sind im Wesentlichen kreisförmig.

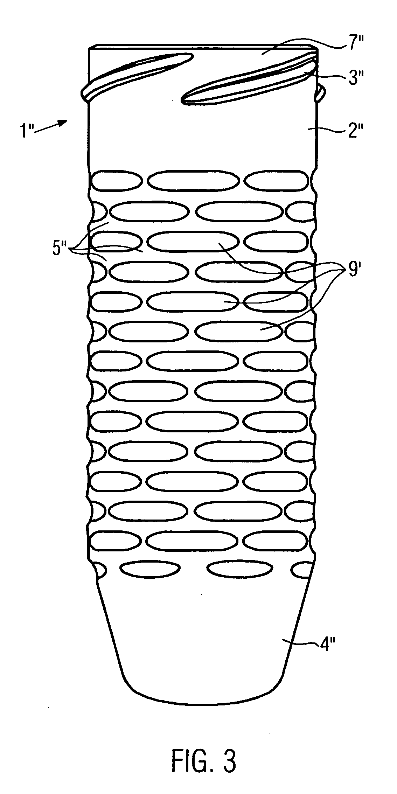

Figur 3 zeigt noch eine weitere Ausführungsform eines Dentalimplantats 1". Im Folgenden werden die Unterschiede zu den beiden bereits beschriebenen Dentalimplantaten aufgezeigt. Das Dentalimplantat 1" umfasst wiederum einen Grundkörper 2". An dem äußeren Ende 4" des Grundkörpers 2" ist ein Gewinde 3" angeordnet. Das Gewinde 3" ist mehrgängig ausgebildet und umfasst vier Gewindegänge. Jeder der Gewindegänge erstreckt sich über etwa ein Viertel des Umfanges des Dentalimplantates 1 ". Auch das Gewinde 3" ist vorzugsweise als Furchgewinde oder selbstschneidendes Gewinde ausgebildet. Das Implantat 1 " kann dann einfach in eine Bohrung im Kieferknochen eingeschoben und durch eine Vierteldrehung präzise fixiert werden.

An der Oberfläche des Grundkörpers 2" sind konkave Einbuchtungen 9' ausgebildet. Die konkaven Einbuchtungen 9' beginnen kurz unterhalb des Gewindes 3" und erstrecken sich bis fast zum inneren Ende 4" des Grundkörpers 2". Die konkaven Einbuchtungen 9' sind in etwa elliptisch und weisen am Übergang zur Oberfläche des Grundkörpers 2" abgerundete Kanten auf. Dadurch wird das Einführen des Implantats 1" in eine Bohrung im Kieferknochen und das Anwachsen von Kieferknochen erleichtert. Die konkaven Einbuchtungen 9' sind auf Kreislinien angeordnet, wobei die Kreislinien um dem Umfang des Grundkörpers 2" verlaufen und jeweils eine Ebene aufspannen, die senkrecht zur Längsachse des Implantats 1" und somit auch zur Längsachse des Grundkörpers 2" verläuft. Die konkaven Einbuchtungen 9' auf zwei benachbarten Kreislinien sind versetzt zueinander angeordnet, so dass eine dichte Anordnung der konkaven Einbuchtungen 9' auf der Oberfläche des Grundkörpers 2" erreicht wird. Die benachbarten Einbuchtungen 9' sind beabstandet zueinander, so dass jeweils zwischen den Einbuchtungen 9' auf nebeneinanderliegenden Kreislinien ein umlaufender kreisförmiger Steg 5" ausgebildet wird. Diese Stege 5" führen, wie auch in den beiden vorherigen Ausführungsbeispielen, dazu, dass das Implantat sich nicht selbst aus dem Kieferknochen herausschrauben kann, wie dies bei Schraubimplantaten vorkommen kann.

Der Grundkörper 2" des Dentalimplantates 1" ist wieder im Wesentlichen zylindrisch ausgebildet. Im unteren Fünftel des Dentalimplantats nimmt der Durchmesser des Grundkörpers 2 ab, so dass sich der Grundkörper 2 nach unten, d. h. zum inneren Ende 4" hin, konisch verjüngt. Dadurch wird das Einsetzen des Dentalimplantats 1" in eine Bohrung im Kieferknochen erleichtert.

Auch das Dentalimplantat 1" kann zusätzlich zu den konkaven Einbuchtungen 9' Mikrorauhigkeiten an der Oberfläche des Grundkörpers 2" aufweisen, um das Anwachsen von Kieferknochen zu erleichtern.

## Patentansprüche

1. Zahnimplantat (1; 1'; 1") mit einem Grundkörper (2; 2'; 2") und einem an einem äußeren Ende (7; 7'; 7") des Grundkörpers, d.h. an dem Ende, das in einem eingesetzten Zustand des Zahnimplantats in den Mundraum zeigt, angeordneten Gewinde (3; 3'; 3"), wobei in dem Bereich des Grundkörpers, der kein Gewinde aufweist, ringförmige Stege (5; 5'; 5") und/oder ringförmige Vertiefungen (6; 6'; 9; 9') ausgebildet sind, **dadurch gekennzeichnet, dass** die Länge des Bereichs des Grundkörpers (2; 2'; 2"), an dem das Gewinde (3; 3'; 3") angeordnet ist, etwa einem Zehntel der Länge des gesamten Grundkörpers entspricht, das Gewinde (3; 3'; 3") mehrgängig ausgebildet ist und die Länge der einzelnen Gewindegänge kleiner ist als der Umfang des Grundkörpers (2; 2'; 2") und vorzugsweise etwa ein Viertel des Umfangs des Grundkörpers (2; 2'; 2") beträgt.

2. Implantat nach Anspruch 1, **dadurch gekennzeichnet, dass** die ringförmigen Stege (5; 5'; 5") und/oder Vertiefungen (6, 6') geschlossen sind.

3. Implantat nach Anspruch 1 oder 2, **dadurch gekennzeichnet, dass** der Bereich des Grundkörpers (2; 2'; 2"), der kein Gewinde aufweist, als Schiebesitz ausgebildet ist.

4. Implantat nach einem der Ansprüche 1 bis 3, **dadurch gekennzeichnet, dass** die ringförmigen Stege (5; 5'; 5") und/oder Vertiefungen (6; 6'; 9; 9') an dem von dem Gewinde abgewandten Ende (4; 4'; 4") des Grundkörpers angeordnet sind.

5. Implantat nach einem der Ansprüche 1 bis 4, **dadurch gekennzeichnet, dass** die ringförmigen Vertiefungen (6; 6'; 9') benachbart zueinander liegen, so dass sie die Stege (5, 5', 5") ausbilden.

6. Implantat nach einem der Ansprüche 1 bis 5, **dadurch gekennzeichnet, dass** die Vertiefungen als ringförmige Rillen (6; 6') und/oder als konkave Einbuchtungen (9; 9') ausgebildet sind.

7. Implantat nach Anspruch 6, **dadurch gekennzeichnet, dass** die konkaven Einbuchtungen (9; 9') auf ringförmigen Linien liegen.

8. Implantat nach Anspruch 7, **dadurch gekennzeichnet, dass** die konkaven Einbuchtungen (9') auf zwei nebeneinanderliegenden ringförmigen Linien versetzt zueinander angeordnet sind.

9. Implantat nach einem der Ansprüche 1 bis 8, **dadurch gekennzeichnet, dass** das innere Ende (4; 4'; 4") des Grundkörpers (2; 2'; 2"), d.h. das Ende, das im eingesetzten Zustand des Grundkörpers im Kiefer angeordnet ist, abgerundet ist.

10. Implantat nach einem der Ansprüche 1 bis 9, **dadurch gekennzeichnet, dass** es aus Keramik, insbesondere Zirkoniumoxid oder Aluminiumoxid oder aus einer Kombination dieser beiden Oxidkeramiken, besteht.

11. Implantat nach einem der Ansprüche 1 bis 10, **dadurch gekennzeichnet, dass** die Oberfläche des Grundkörpers (2; 2'; 2") Mikrorauhigkeiten aufweist.

## Claims

1. Dental implant (1; 1'; 1") having a base member (2; 2'; 2") and a thread (3; 3'; 3") arranged at an outer end (7; 7'; 7") of said base member, i.e. at the end which when the dental implant is inserted faces towards the oral cavity, where in the region of said base member having no thread, annular ridges (5; 5'; 5") and / or annular recesses (6; 6'; 9; 9') are formed, **characterized in that** the length of the section of said base member (2; 2'; 2") on which the thread (3; 3'; 3") is disposed corresponds to approximately one tenth of the length of said entire base member, that said thread (3; 3'; 3") is formed with multiple entries and that the length of the individual thread turns is less than the perimeter of said base member (2; 2'; 2") and preferably is about one quarter of the perimeter of said base member (2; 2'; 2").

2. Implant according to claim 1, **characterized in that** said annular ridges (5; 5'; 5") and / or recesses (6, 6') are closed.

3. Implant according to claim 1 or 2, **characterized in that** said section of said base member (2; 2'; 2") having no thread is formed as a sliding seat.

4. Implant according to one of the claims 1 to 3, **characterized in that** said annular ridges (5; 5'; 5") and / or recesses (6; 6'; 9; 9 ') are disposed on the end (4; 4'; 4") of said base member facing away from said thread.

5. Implant according to one of the claims 1 to 4, **characterized in that** said annular recesses (6; 6'; 9') are located adjacent to each other such that they form ridges (5; 5'; 5").

6. Implant according to one of the claims 1 to 5, **characterized in that** said recesses are formed as annular grooves (6; 6') and / or concave indentations (9; 9').

7. Implant according to claim 6, **characterized in that** said concave indentations (9; 9') are located on annular lines.

8. Implant according to claim 7, **characterized in that** said concave recesses (9') are arranged offset to each other on two adjacent annular lines.

9. Implant according to one of the claims 1 to 8, **characterized in that** said inner end (4; 4'; 4") of said base member (2; 2'; 2") is rounded, i.e. the end which is disposed in said jaw bone when said base member is inserted.

10. Implant according to one of the claims 1 to 9, **characterized in that** it is made from ceramic, in particular zirconium oxide or aluminum oxide or a combination of both oxide ceramics.

11. Implant according to one of the claims 1 to 10, **characterized in that** said surface of said base member (2; 2'; 2") comprises microroughnesses.

## Revendications

1. Implant dentaire (1; 1'; 1") comprenant un corps de base (2; 2'; 2") et un filetage (3; 3' ; 3") agencé à une extrémité extérieure (7; 7'; 7") du corps de base, c'est-à-dire l'extrémité, qui dans l'état implanté de l'implant dentaire est dirigée vers la cavité buccale, des nervures de forme annulaire (5; 5'; 5") et/ou des creux de forme annulaire (6; 6'; 9; 9') étant formés dans la zone du corps de base, qui ne présente pas de filetage, **caractérisé en ce que** la longueur de la zone du corps de base (2; 2'; 2") sur laquelle est agencé le filetage (3; 3'; 3") correspond environ à un dixième de la longueur totale du corps de base, le filetage (3; 3'; 3") est d'une configuration à filets multiples, et la longueur des filets individuels est inférieure à la longueur de la circonférence du corps de base (2; 2'; 2") et vaut de préférence un quart de la circonférence du corps de base (2; 2'; 2").

2. Implant selon la revendication 1, **caractérisé en ce que** les nervures de forme annulaire (5; 5'; 5") et/ou les creux de forme annulaire (6; 6') sont fermés.

3. Implant selon la revendication 1 ou la revendication 2, **caractérisé en ce que** la zone du corps de base (2; 2'; 2"), qui ne présente pas de filetage, est réalisée en tant qu'ajustement coulissant.

4. Implant selon l'une des revendications 1 à 3, **caractérisé en ce que** les nervures de forme annulaire (5; 5'; 5") et/ou les creux de forme annulaire (6; 6'; 9; 9') sont agencés à l'extrémité (4; 4'; 4") du corps de base, qui est opposée à celle où se trouve le filetage.

5. Implant selon l'une des revendications 1 à 4, **caractérisé en ce que** les creux de forme annulaire (6; 6'; 9') sont placés de manière voisine les uns des autres, de sorte qu'ils forment les nervures (5; 5'; 5").

6. Implant selon l'une des revendications 1 à 5, **caractérisé en ce que** les creux sont réalisés en tant que stries de forme annulaire (6; 6') et/ou d'empreintes concaves (9; 9').

7. Implant selon la revendication 6, **caractérisé en ce que** les empreintes concaves (9; 9') se situent sur des lignes de forme annulaire.

8. Implant selon la revendication 7, **caractérisé en ce que** les empreintes concaves (9') sont agencées de manière à être mutuellement décalées les unes par rapport aux autres sur deux lignes de forme annulaire voisines.

9. Implant selon l'une des revendications 1 à 8, **caractérisé en ce que** l'extrémité intérieure (4; 4'; 4") du corps de base (2; 2'; 2"), c'est-à-dire l'extrémité, qui dans l'état implanté du corps de base se situe dans la mâchoire, est arrondie.

10. Implant selon l'une des revendications 1 à 9, **caractérisé en ce qu'**il est réalisé en céramique, notamment en oxyde de zirconium ou en oxyde d'aluminium ou en une combinaison de ces deux céramiques d'oxydes.

11. Implant selon l'une des revendications 1 à 10, **caractérisé en ce que** la surface du corps de base (2; 2'; 2") présente des microrugosités.
